# FASCICULE DE BREVET EUROPEEN

(11) **EP 1 453 559 B1**
(45) Date de publication et mention de la délivrance du brevet: **19.12.2007**
(21) Numéro de dépôt: 02783392.0
(22) Date de dépôt: 20.11.2002
(51) Int. Cl.: A61M 1/16, A61M 1/28

(54) **DISPOSITIF D'EPURATION INTRA-ET EXTRACORPORELLE**
VORRICHTUNG ZUR INTRA- UND EXTRAKORPORALEN REINIGUNG
DEVICE FOR INTRACORPOREAL AND EXTRACORPOREAL PURIFICATION

(30) Priorité: 26.11.2001 EP 01128017
(43) Date de publication de la demande: 08.09.2004
(73) Titulaire: Infomed S.A., 1227 Genève (CH)
(72) Inventeur: FAVRE, Olivier, Claude, 1208 Genève (CH)
(74) Mandataire: Micheli & Cie SA
(86) Numéro de dépôt international: PCT/IB2002/004826
(87) Numéro de publication internationale: WO 2003/045471

(56) Documents cités:
- EP-A- 0 928 615
- EP-A- 1 095 666
- US-A- 4 190 047
- US-A- 5 783 072

## Description

La présente invention se rapporte à un dispositif d'épuration de patients souffrant d'insuffisance rénale, cette épuration ayant pour but de nettoyer le corps des patients en en retirant des éléments indésirables, notamment l'urée et l'eau.

Elle peut être réalisée par différentes méthodes connues, les principales étant la dialyse péritonéale, l'hémodialyse et l'hémofiltration. La dialyse péritonéale consiste à remplir le péritoine du patient d'une solution qui se pollue par migration de molécules à travers la membrane naturelle du péritoine puis est retirée en emmenant ces molécules avec elle. Pour augmenter l'efficacité de la méthode plusieurs cycles peuvent être réalisés à la suite. Les autres méthodes consistent à épurer le sang en le faisant circuler à l'extérieur du corps d'un côté d'une membrane artificielle semi-perméable, certains constituants traversant cette membrane sous l'effet, pour l'hémodialyse, d'un gradient de concentration ou, pour l'hémofiltration, de pression. Dans certains cas on combine deux méthodes comme par exemple l'hémodiafiltration qui réalise simultanément une hémodialyse et une hémofiltration. Dans tous les cas le patient doit être connecté plusieurs fois par semaine, typiquement trois à six fois, à l'appareil d'épuration pour des séances d'une durée de quelques heures.

Depuis les années 1970 il existe des appareils qui permettent de réaliser ces différentes méthodes d'épuration. La plupart d'entre eux sont conçus uniquement pour l'hémodialyse, d'autres permettent de réaliser soit l'hémofiltration ou l'hémodialyse soit la combinaison de ces deux méthodes, l'hémodiafiltration. Les appareils de dialyse péritonéale sont normalement plus simples et limités à certaines variantes de ce traitement.

La dialyse péritonéale est une méthode qui s'applique surtout aux patients désireux d'êtres traités à domicile, ce qui impose aux appareils correspondants une conception garantissant la sécurité du patient et simultanément une maniabilité facile. Dans sa version la plus simple la gestion des fluides est faite manuellement par le patient lui-même. Des appareils existent qui permettent une gestion automatique des fluides, diminuant ainsi la charge de travail du patient et augmentant l'efficacité du traitement mais retirant en contrepartie du patient des quantités importantes de "grosses molécules", notamment des protéines, ce qui n'est pas désirable. La dialyse péritonéale a également l'inconvénient de ne permettre de retirer que de faibles quantités de l'eau absorbée par le patient entre deux séances de dialyse. L'efficacité de la dialyse péritonéale et des appareils correspondants est limitée par la capacité de la membrane naturelle du péritoine, les patients dont le besoin d'épuration augmente au cours du temps doivent alors recourir à une autre méthode plus efficace et normalement changer d'appareil ainsi que de lieu de traitement, ce qui est un inconvénient important.

Actuellement, les séances d'hémodialyse et d'hémofiltration sont essentiellement réalisés dans des centres hospitaliers. Depuis les années 1970 des appareils d'hémodialyse et d'hémofiltration pour l'utilisation à domicile sont disponibles. On peut notamment citer les appareils de la société AKSYS (USA) qui ont été spécifiquement développés durant les années 1995 à 2000 dans le but de couvrir les besoins spécifiques de l'hémodialyse et de l'hémofiltration à domicile. Les appareils d*'*hémofiltration de la société GAMBRO ont quant à eux été utilisés depuis les années 1980 pour réaliser l'hémofiltration à domicile.

Afin de décrire l'état de la technique plus en détail, on peut constater que les appareils de dialyse péritonéale possèdent généralement deux pompes, l'une pour injecter du liquide de dialyse, le dialysat, et l'autre pour retirer ce liquide une fois qu'il a été pollué. Des moyens de gestion des volumes entrant et sortant du patient permettent de contrôler le poids de ce dernier, c'est-à-dire d'éviter une surcharge du patient en dialysat et de contrôler le volume d'eau qui lui est retiré. Le dialysat stérile est dans la plupart des cas injecté à partir de poches. L'adéquation de son débit avec les capacités du site du patient est contrôlée grâce à des capteurs de pression situés sur les tubulures d'injection et d'extraction du dialysat.

Les appareils d'hémodialyse possèdent généralement trois pompes, une pour faire circuler le sang à l'extérieur du corps du patient, une pour faire circuler un fluide, appelé dialysat ou solution d'échange, à contre-courant du sang de l'autre côté de la membrane semi-perméable et une pour contrôler le volume de fluide extrait du sang à travers ladite membrane, le paramètre de réglage étant habituellement le gradient de pression communément appelé pression transmembranaire, à travers ladite membrane. Le dialysat est généralement fabriqué en continu par l'appareil d'hémodialyse à partir d'eau et d'acide ou de poudre de sels. Dans certains cas le dialysat est directement injecté à partir de poches. Des moyens de mesure des débits de dialysat et de fluide extrait sont utilisés pour assurer la qualité du traitement et le contrôle du poids du patient. Des moyens permettant de prévenir l'injection d'air et de détecter la déconnexion du circuit de sang ou d'autres erreurs similaires sont également prévus.

Les appareils d'hémofiltration comportent généralement trois ou quatre pompes, une pour faire circuler le sang à l'extérieur du patient, une pour extraire une solution polluée appelée ultrafiltrat à travers la membrane et une ou deux pour injecter une solution de substitution. Lorsque deux pompes sont utilisées à ce dernier effet, l'une dilue le sang avant son contact avec la membrane semi-perméable (prédilution) et l'autre après (postdilution). Des moyens de mesure et de contrôle des volumes d'ultrafiltrat et de solution de substitution complètent ces appareils. Ce sont le plus souvent des balances ou des chambres de volume connu associées à une unité de contrôle qui, entre autre, veille à ce que le poids du patient soit maîtrisé lors du traitement. La solution de substitution est injectée à partir de poches, certains systèmes permettant cependant une génération continue de liquide de substitution à partir d'eau et d'acides ou de sels. Dans ce cas, le passage à travers des filtres supplémentaires est nécessaire pour rendre la solution stérile et apyrogène avant son injection au patient.

Si aucune solution de substitution n'est injectée, le traitement consiste uniquement à retirer de l'ultrafiltrat et est appelé ultrafiltration.

Dans un appareil d'épuration extracorporelle du sang conventionnel l'unité de calcul qui pilote l'appareil assure la circulation du sang et celle des volumes extraits et injectés dans le système en fonction des règles de fonctionnement établies pour le traitement à réaliser, des spécifications de l'opérateur et des informations lues par les différents moyens de mesure de l'appareil, notamment la circulation effective du sang. Par exemple, lorsque le débit du sang est nul suite à une alarme d'air ou de pression, les débits de la solution d'échange et de la solution polluée doivent également être nuls. Dans un tel appareil, les débits de la solution d'échange et de la solution polluée sont pilotés en fonction de la circulation effective du sang. Ainsi, si la solution polluée et la solution d'échange étaient directement injectées au patient soit extraites du patient, comme c'est le cas pour la dialyse péritonéale dans sa réalisation de base, l'unité de calcul ne pourrait fournir un débit non nul du fait que les critères nécessaires de la circulation du sang ne seraient pas satisfaits.

Les appareils d'épuration extracorporelle du sang sont généralement prévus pour extraire le sang depuis un endroit du patient et le retourner à un autre endroit. La circulation du sang est ainsi continue. Dans certains cas les appareils fonctionnent en mode alterné extraction/retour avec une connexion unique au patient. On ajoute alors des chambres d'expansion sur le circuit sang et une pompe ou des clamps supplémentaires, piloté par l'unité de calcul, sur l'appareil. Cette complexité supplémentaire de l'appareil est parfois préférée car elle réduit les risques d'infection du patient en réduisant d'un facteur 2 les sites d'abord vasculaire. En dialyse péritonéale on a au contraire toujours un site unique qui est utilisé alternativement pour injecter et extraire le dialysat.

Dans les traitements agissant sur le sang les débits de la solution polluée et de la solution d'échange sont continus aussi longtemps que la pompe à sang fonctionne. Au contraire, en dialyse péritonéale l'appareil injecte dans un premier temps le dialysat, attend que le dialysat se pollue à l'intérieur du patient puis retire la solution polluée. Les cycles de solution polluée et de solution d'échange sont donc alternés et séparés d'une période d'arrêt lors de la dialyse péritonéale.

Un appareil procédant par circulation extracorporelle du sang possède de plus des éléments de sécurité que n'incorporent pas les appareils de dialyse péritonéale. On peut notamment citer les moyens permettant d'éviter l'injection d'air au patient, souvent composés de pièges à bulles d'air, de détecteurs d'air et de clamps, et les moyens permettant de détecter une éventuelle déconnexion des tubulures de circulation du sang, habituellement un capteur de pression placé en amont de la pompe à sang et un autre placé sur la ligne de retour de sang. Si certains systèmes de dialyse péritonéale comprennent des moyens afin d'éviter l'injection d'air, la déconnexion des tubulures n'est pas considérée comme un problème majeur dans ce cas, car elle n'implique pas un danger élevé pour le patient. Une déconnexion présente cependant des inconvénients pour l'hygiène du patient, augmentant ainsi le risque d'infection du site et souillant son environnement, par exemple son lit. Il paraît donc opportun de protéger le patient contre ces deux inconvénients.

Un appareil d'épuration extracorporelle est décrit dans EP-A-1 095 666.

Vu de ce qui précède, un dispositif d'épuration extracorporelle conventionnel prévu pour épurer le sang ne peut donc pas être utilisé dans un mode de dialyse péritonéale.

L'intérêt de la dialyse péritonéale est qu'elle est particulièrement bien adaptée à l'utilisation par le patient lui-même à son domicile du fait qu'elle ne nécessite pas d'extraire le sang du patient et évite de ce fait les risques y relatifs comme par exemple la perte de sang par ouverture involontaire d'un conduit, l'embolie gazeuse par injection d'air ou la coagulation du sang au contact des conduits et de la membrane semi-perméable. Nécessitant peu de matériel elle est de plus économique.

La dialyse péritonéale comporte certains inconvénients, notamment l'utilisation du péritoine, membrane naturelle, comme membrane semi-perméable ce qui limite les capacités d'épuration de la dialyse péritonéale laquelle n'est en conséquence pas applicable à tous les patients. Les capacités rénales d'un patient utilisant cette méthode peuvent diminuer dans le temps obligeant l'utilisation d'une autre méthode, habituellement l'hémodialyse. La dialyse péritonéale est ainsi surtout utilisée au début de l'insuffisance rénale alors que les reins fonctionnent encore partiellement fournissant ainsi un complément d'épuration. Une difficulté similaire de la dialyse péritonéale est que la capacité de retirer de l'eau du patient est limitée menant à une fréquente surcharge hydrique du patient et qu'elle nécessite l'adjonction de glucose dans le dialysat ce qui complique sa fabrication et augmente le coût de ce dernier. Les quantités de protéines perdues à chaque séance sont importantes et parfois difficiles à compenser. De plus, le site d'injection et d'extraction du dialysat dans le corps du patient donne lieu à des épisodes infectieux assez fréquents. Dans ce cas le patient est traité dans un centre hospitalier par hémodialyse ou hémofiltration en attendant que l'infection soit réduite et que le site soit de nouveau utilisable. Souvent le patient restera par la suite traité dans le centre hospitalier car dans l'intervalle sa défaillance rénale peut s'aggraver, son infection être telle qu'elle empêche de placer un nouveau site ou la confiance du patient être atteinte au point qu'il souhaite renoncer à se soigner lui-même.

Ces limites de la dialyse péritonéale font que les patients sont traités en moyenne seulement deux à trois ans avec cette méthode avant d'être épurés dans un centre hospitalier par hémodialyse. L'investissement lié à la formation du patient qui a dû apprendre à utiliser son appareil est alors perdu et sa qualité de vie liée à la souplesse des horaires et à la fréquence des traitements pouvant être réalisés à domicile décroît fortement. Le patient doit souvent complètement modifier son emploi du temps et reconsidérer ses capacités à travailler.

Compte tenu du fait que les patients débutent normalement leur traitement à domicile à un stade où le besoin d'épuration est relativement faible et donc avec un appareil pour la dialyse péritonéale, les coûts du changement d'appareils et l'inconvénient d'un re-apprentissage afin de pouvoir continuer le traitement à domicile avec une méthode de traitement plus efficace, alors l'épuration extracorporelle du sang, constituent des désavantages non-négligeables.

Les brevets US 4190047 et US 4338190 décrivent des appareils de dialyse péritonéale dans lesquels le dialysat en contact avec le péritoine est épuré pendant la séance à travers une membrane semi-perméable. Ainsi, comme pour une machine d'hémodialyse, on retrouve dans les deux inventions une première boucle de circulation pour le dialysat et une seconde boucle de circulation dans laquelle ce n'est ici plus du sang mais également du dialysat qui est circulé et introduit dans le péritoine du patient. Les deux boucles sont séparées par ladite membrane semi-perméable afin d'épurer le dialysat se trouvant dans la seconde boucle en contact avec le péritoine, ce qui permet de réduire les pertes des protéines en recyclant le dialysat.

Le brevet US 5141493 décrit un système de dialyse péritonéale composé également de deux boucles de circulation de dialysat séparées par une membrane semi-perméable mais équipée de plus d'une jonction du patient à la seconde boucle contrôlée par une pompe indépendante. Le dialysat contenu dans le corps du patient peut alors se polluer indépendamment du nettoyage du dialysat qui sera utilisé lors du prochain cycle.

Ainsi, si les inventions mentionnées améliorent les appareils de dialyse péritonéale en leur adjoignant des éléments que l'on retrouve dans les appareils d'hémodialyse, elles ne permettent plus de réaliser la dialyse péritonéale dans sa forme de base la plus économique et répandue, soit sans épuration du dialysat dans une seconde boucle de circulation. Surtout, les appareils restent restreint à la dialyse péritonéale, l'idée d'utiliser la seconde boucle dans un autre mode d'opération, la circulation extracorporelle du sang, et ainsi de réaliser avec le même appareil une hémodialyse ou une hémofiltration d'une part et une dialyse péritonéale d'autre part n'est pas présente. Les inventions mentionnées apportent toutes une complexité supplémentaire en matériel et en coût, notamment par l'utilisation de filtres artificiels, sans pour autant résoudre les problèmes les plus graves de la dialyse péritonéale, notamment le changement de méthode et d'appareil de traitement rendu nécessaire par exemple par l'infection des sites d'accès à la cavité péritonéale du patient.

En effet, aucun des appareils de dialyse péritonéale existants ne permet de réaliser une épuration par circulation extracorporelle du sang comme l'hémodialyse ou l'hémofiltration ce qui permettrait d'éviter les inconvénients de la dialyse péritonéale décrits ci-dessus et autoriserait le patient à rester à son domicile lors d'infection du site ou de baisse de sa fonction rénale. L'épuration extracorporelle du sang complémentaire permettrait également de retirer le surplus d'eau du patient traité par dialyse péritonéale en pratiquant une ultrafiltration. Par rapport à la méthode classique de la dialyse péritonéale, l'utilisation d'un filtre supplémentaire permet d'épurer la solution polluée afin de l'injecter à nouveau dans le corps du patient réduisant ainsi les pertes des protéines.

Cette description plus détaillée des différents dispositifs connus montre que les caractéristiques techniques des appareils varient pour chaque type de traitement et que plusieurs variantes existent. Certains appareils permettent de réaliser plusieurs types de traitements et d'autres de combiner ces types par exemple pour réaliser de l'hémodiafiltration avec génération du dialysat et du liquide de substitution en continu, d'autres appareils l'injectant à partir de poches. Toutefois aucun appareil n'a été proposé à ce jour qui permettrait de réaliser d'une part la dialyse péritonéale dans sa forme de base et d'autre part une épuration par circulation extracorporelle du sang, ce qui est remarquable compte tenu de l'application de toutes ces méthodes de traitement depuis des décennies.

Le but de la présente invention est de remédier, au moins en partie, aux inconvénients précités et de réaliser un dispositif d*'*épuration intra- et extracorporelle du patient permettant la combinaison des différentes méthodes de traitement actuellement connues, notamment la dialyse péritonéale, l'hémofiltration et l'hémodialyse, et offrant de plus la possibilité de l'application de l'ultrafiltration afin de soulager le surcharge hydrique du patient sans ou avec adjonction de glucose dans le dialysat ainsi que des moyens pour l'épuration de la solution polluée afin de la réinjecter dans le corps en évitant au patient des pertes importantes des protéines.

A cet effet, la présente invention a pour objet un dispositif d'épuration intra- et extracorporelle comportant les caractéristiques énoncées à la revendication 1 et aux revendications dépendantes.

L'invention sera mieux comprise à l'aide des dessins annexés qui illustrent schématiquement et à titre d'exemple une forme d'exécution du dispositif selon la présente invention.

La figure 1 représente schématiquement une forme d'exécution de l'invention permettant de réaliser un traitement basé sur l'épuration extracorporelle du sang comme l'hémodialyse ou l'hémofiltration.

La figure 2 illustre une forme d'exécution de l'invention permettant de réaliser un traitement basé sur l'utilisation de la cavité péritonéale du patient comme la dialyse péritonéale.

La figure 3 représente schématiquement une forme d'exécution de l'invention permettant de réaliser un traitement basé sur l'utilisation de la cavité péritonéale du patient comme la dialyse péritonéale permettant simultanément d'épurer la solution polluée pour la réinjecter, avec les molécules larges et notamment les protéines, dans le corps du patient.

La description détaillée qui suit expliquera à l'aide des dessins susmentionnés la conception, le fonctionnement et les avantages d'un dispositif selon l'invention.

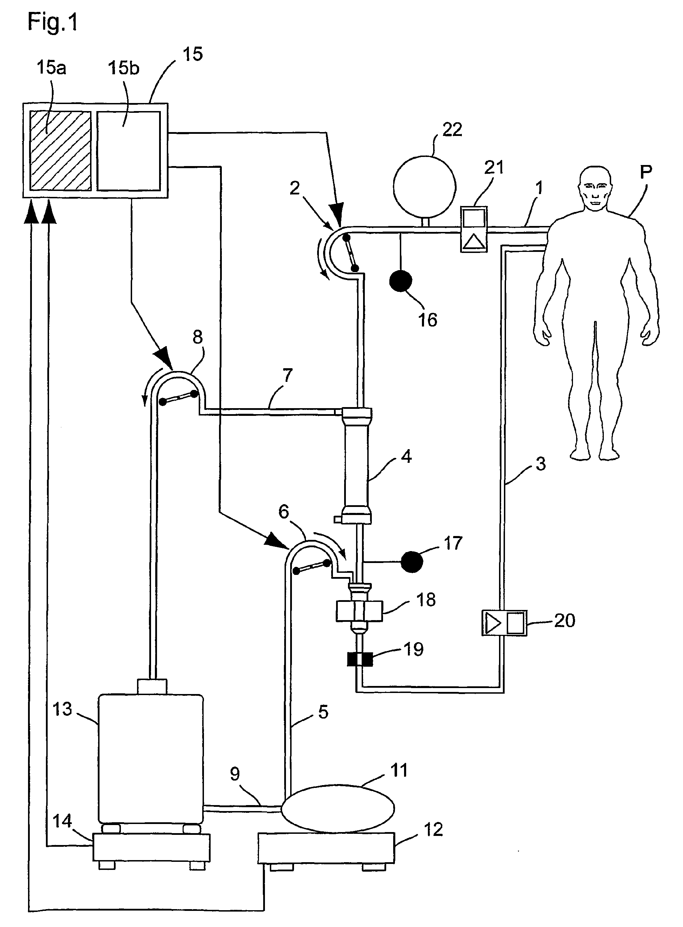

Un dispositif d'épuration selon l'invention comporte, afin de permettre un premier mode d'opération du dispositif pour l'épuration extracorporelle du sang, des moyens de circulation extracorporelle de sang, ce circuit extracorporelle comprenant un conduit d'extraction 1, un moyen de commande de débit 2 comme une pompe pour extraire le sang du corps du patient P, un conduit de retour 3 pour ramener le sang épuré dans le corps du patient P et un élément d'épuration 4, qui est placé entre le conduit d'extraction 1 et le conduit de retour 3 et qui possède une membrane semi-perméable permettant de réaliser l'épuration du sang. Un piège à bulles 18 permet de retenir les bulles d'air, un détecteur d'air 19 assure que l'air ne pourra être retourné au patient, un clamp veineux 20 est fermé en cas de détection d'air empêchant ainsi l'injection d'air au patient, et des capteurs de pression 16 et 17 permettent de détecter une éventuelle déconnexion du circuit de circulation du sang. Ces éléments sont illustrés schématiquement à la figure 1.

Un conduit d'introduction 5 de solution d'échange possédant un moyen de commande de débit 6, habituellement une pompe ou un clamp, relie un réservoir de solution d'échange 11 au circuit de circulation extracorporelle 1, 2, 3, 4. Le réservoir de solution d'échange 11 peut être remplacé par un moyen de génération en continu de cette solution à partir d'eau et d'acide ou de poudre de sels. Le conduit d'introduction 5 peut être connecté au conduit d'extraction 1 (en mode de prédilution), au conduit de retour 3 (en mode de postdilution), à l'élément d'épuration 4 du côté de la membrane semi-perméable où le sang ne circule pas (en mode de dialyse) ou selon une combinaison quelconque de ces différents modes. Le circuit de la solution d'échange est complété par des moyens de mesure 12 de la masse de solution d'échange, par exemple des capteurs de force, de volume ou de débit.

Un conduit d'évacuation 7 de la solution polluée possédant un moyen de commande de débit 8, habituellement une pompe ou un clamp, est raccordé à l'élément d'épuration 4 du côté de la membrane semi-perméable où le sang ne circule pas. La solution polluée atteint ainsi des moyens d'évacuation 13, lesdits moyens pouvant être une unité de recueil temporaire, poche ou bidon ou une conduite permettant une évacuation en continu. Le circuit d'évacuation de la solution polluée est complété par des moyens de mesure 14 de la masse de solution évacuée, par exemple des capteurs de force, de volume ou de débit.

Un module de commande 15 comprend des moyens de calcul 15a qui servent à piloter les moyens de commande de débit 2, 6, 8 en fonction des paramètres déterminant les quantités respectives de sang, de solution d'échange et de solution polluée. Les indications fournies par les moyens de mesure 12 et 14 sont utilisées par les moyens de calcul 15a pour déterminer les masses entrantes et sortantes du patient. Différentes méthodes de pilotage existent et sont décrites dans la littérature. Les paramètres sont généralement des consignes inscrites par l'opérateur et des valeurs lues par des capteurs comme par exemple le nombre de tours des pompes, les masses mesurées par des balances ou le nombre de cycles remplissage/vidange d'une éprouvette de volume calibré.

Les composants habituels des dispositifs d'épuration extracorporelle qui n'interviennent pas directement dans le champ de la présente invention ne sont pas représentés. Ce sont notamment le détecteur de fuite de sang, les capteurs de pression transmembranaire, le réchauffeur de sang ou de liquide d'échange et l'interface utilisateur.

Les moyens de commande de débit 2, 6 et 8 sont typiquement des pompes péristaltiques, des pompes à galets ou des clamps.

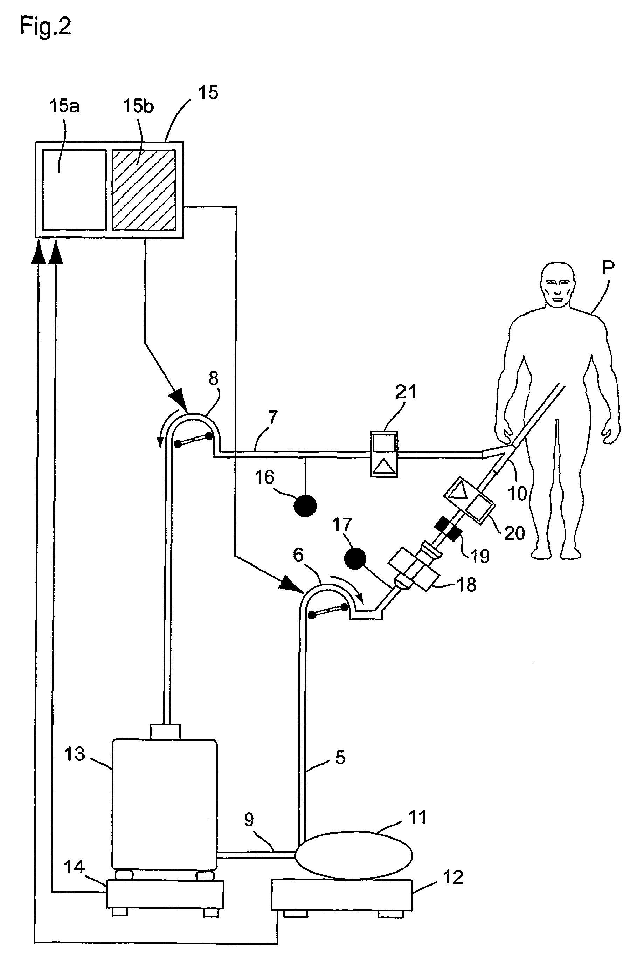

Un dispositif d'épuration selon la présente invention permet également, comme illustré schématiquement à la figure 2 et afin de réaliser un second mode d'opération du dispositif pour l'épuration intracorporelle du patient, c'est-à-dire la dialyse péritonéale dans sa forme de base, d'une part de modifier la tubulure de circulation des fluides en en retirant le circuit de circulation extracorporelle 1, 2, 3, 4 du sang et en ajoutant un élément spécifique 10 en forme de "Y" raccordé à la conduite d'injection 5 de la solution d'échange, la conduite d'extraction 7 et le site du patient, et d'autre part de gérer le transport des fluides par un seul site aussi bien que par deux sites en comprenant, en plus de la première unité de calcul 15a, une deuxième unité de calcul 15b capable de piloter les débits de la solution d'échange et de la solution polluée non plus de manière simultanée mais de manière alternative et indépendante de la circulation du sang. A cet effet, les moyens de commande de débit 6 et 8 sont pilotés en fonction de divers paramètres définissants la forme des débits en fonction du temps et le délai séparant le cycle d'injection de celui d'extraction et des valeurs lues depuis les moyens de mesure 12 et 14. Les capteurs de pression 16 et 17, placés ici sur le conduit d'introduction 5 et le conduit d'évacuation 7, sont dans ce cas utilisés dans deux buts. D'une part pour vérifier que le site permet d'assurer les débits souhaités et d'autre part pour détecter une éventuelle déconnexion des circuits. Dans une variante, un seul capteur situé de préférence sur l'élément spécifique 10 peut fournir les mêmes indications.

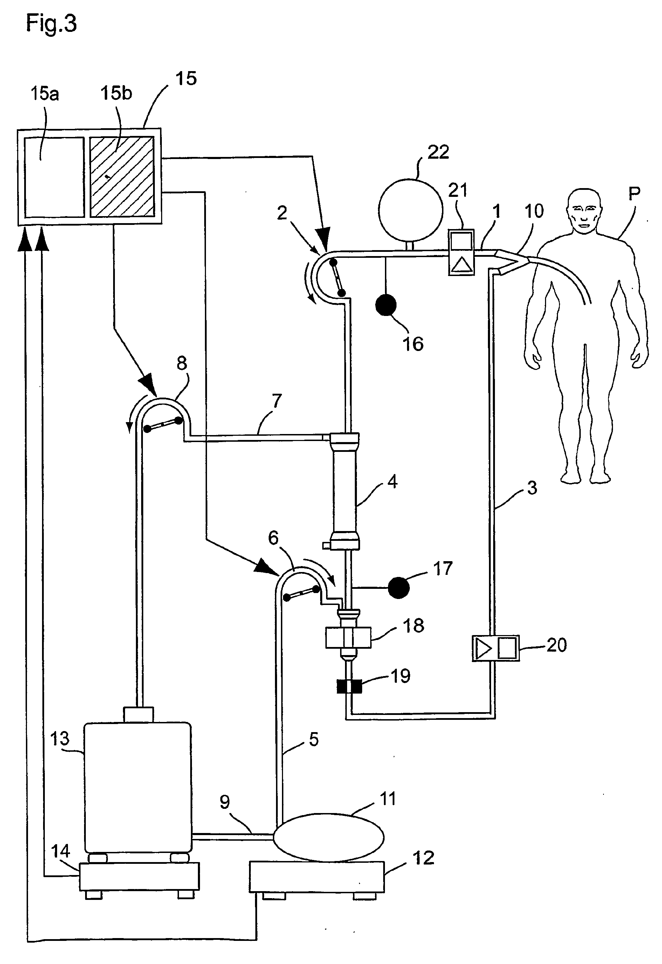

Un dispositif d'épuration selon l'invention permet dans encore un autre mode d'opération afin de réaliser la dialyse péritonéale affinée, qui est illustré à la figure 3, de faire circuler dans le circuit extracorporelle, au lieu de sang, la solution d'échange, qui est ainsi injectée dans le péritoine du patient, puis extraite et ensuite épurée à l'aide des moyens d'épuration 4 de manière à retenir les solutés de grandes dimensions dont la déplétion dans l'application habituelle de la dialyse péritonéale, où le dialysat pollué est entièrement évacué, nuit à l'état de santé du patient. Le dispositif comporte à cet effet deux clamps 20, 21 et une chambre d'expansion 22 permettant de faire circuler le dialysat en mode alterné en utilisant un site unique. L'élément spécifique 10 ou une aiguille unique peuvent être intégrés dans la tubulure servant normalement de circuit extracorporelle 1, 2, 3, 4 du sang pouvant ainsi être connecté dans ce mode d'opération à un site unique du péritoine du patient. Dans ce cas, on injecte au départ la solution d'échange dans le péritoine en ouvrant le clamp 20, fermant le clamp 21 et en faisant marcher le moyen de commande de débit 6. La solution d'échange peut alors extraire des éléments non-souhaités du corps du patient à travers la membrane du péritoine pendant une durée prédéterminée. Pour extraire le dialysat pollué, le clamp 20 est fermé et le clamp 21 est ouvert pendant que le moyen de commande de débit 2 est activé, ce qui rempli la chambre d'expansion 22. Pour retourner le dialysat au patient, on ouvre le clamp 20 et ferme le clamp 21 de nouveau et on active le moyen de commande de débit 2 vidant ainsi la chambre 22. Durant cette phase de retour, les pompes 6, 8 sont activées pour épurer le dialysat pollué qui passe dans l'élément d'épuration 4. Les capteurs de pression 16, 17 permettent de détecter la fin des cycles d'extraction ou de retour. La deuxième unité de calcul 15b est agencée de manière à ce qu'elle puisse gérer ce mode d'opération en pilotant les moyens de commande de débit 2, 6, 8 tenant compte des valeurs détectées dans les capteurs de pression 16, 17. Un recyclage de la solution d'échange est donc rendu possible et des pertes importantes des protéines peuvent être évitées par ce dispositif.

Le même dispositif avec une tubulure comme décrit ci-dessus mais le circuit extracorporelle 1, 2, 3, 4 étant connecté, par exemple à l'aide d'une aiguille unique, au circuit sanguin du patient peut être utilisé pour l'épuration extracorporelle du sang en utilisant les éléments comme les clamps 20, 21 et la chambre d'expansion 22 de manière adéquate pour ce mode d'opération. L'invention s'applique donc également aux systèmes comprenant les éléments de circulation extracorporelle de sang à aiguille unique et à ses différentes variantes décrites dans la littérature.

Une forme d'exécution d'un dispositif d'épuration selon la présente invention permet de réaliser un traitement de type ultrafiltration en retirant de l'eau du sang du patient. Dans le mode préféré, le dispositif extrait le sang depuis un abord vasculaire périphérique unique. Comme décrit ci-dessus, la tubulure permet l'utilisation d'une aiguille unique, et les paramètres de l'unité de calcul 15a sont dans ce cas adaptés afin de permettre une circulation sanguine à un débit inférieur à celui utilisé avec des accès vasculaires obtenus avec des cathéters ou fistules.

Un dispositif selon l'invention est donc capable d'effectuer la dialyse péritonéale ainsi que l'hémodialyse ou l'hémofiltration et l' ultrafiltration dans différentes variantes de traitement, réalisant ainsi une épuration intra- et extracorporelle du sang.

Dans tous les cas décrits ci-dessus, le réservoir de la solution d'échange 11 et les moyens d'évacuation 13 peuvent être reliés par un conduit de liaison 9 ou formés d'un composant unique dans le but d'utiliser la solution évacuée comme solution d'échange. En effet, comme il est rare que la solution d'échange soit saturée lors du premier passage dans l'élément d'épuration 4, cette méthode permet une économie de solution d'échange fraîche.

De même, l'invention s'applique aux appareils intégrant ou non la fabrication des solutions d'échange, ainsi qu'aux méthodes combinées comme l'hémodiafiltration ou l'utilisation simultanée de prédilution et postdilution.

Un dispositif selon l'invention peut aussi comporter des moyens pour nettoyer les moyens d*'*épuration 4 et la tubulure. Ces éléments sont alors conservés sur l'appareil et utilisés pour plusieurs traitement consécutifs. Les moyens de nettoyage permettent l'injection d'une substance nettoyante, par exemple de l'acide acétique, et d'une substance de rinçage, par exemple une solution saline, et comportent de plus des moyens de contrôle et de sécurité, par exemple une sonde de mesure du pH du liquide contenu dans le circuit de circulation extracorporelle.

Il est évident que l'idée de la présente invention englobe la possibilité de réaliser un dispositif d'épuration intra- et extracorporelle selon l'invention en ajoutant des pièces de tubulure, de commande, de contrôle et de sécurité ainsi que des moyens de calcul nécessaires à cet effet à un appareil de dialyse péritonéale afin de réaliser avec un tel appareil également l'épuration du sang par hémodialyse, par hémofiltration et/ou par ultrafiltration. Ceci est possible pour toutes les formes d'exécution décrites ci-dessus, sans nécessité de répéter les détails et sans limitation de l'applicabilité de l'invention à ce but.

De même, il est possible de fournir les pièces et/ou les moyens de commande nécessaires pour réaliser un dispositif d'épuration intra- et extracorporelle selon l'invention à partir d'un appareil d'épuration extracorporelle du sang permettant ainsi à celui-ci d'effectuer également la dialyse péritonéale sans ou avec recyclage de la solution d'échange.

Un dispositif d'épuration du corps selon la présente invention permet alors d'effectuer les différentes méthodes d'épuration extracorporelle décrites précédemment, notamment d'une part des dialyses péritonéales et d'autre part les méthodes complémentaires que sont l'hémodialyse, l'hémofiltration et l'ultrafiltration aussi bien que la combinaison de ces méthodes en comportant les circuits adéquats à chacune de ces méthodes de traitement et en comprenant également les moyens de commande et de gestion de fluides correspondants.

L'invention permet alors d'éviter la surcharge hydrique du patient en dialyse péritonéale avec ou sans adjonction de glucose dans le dialysat.

De plus, les pertes gênantes des protéines peuvent être évitées par le recyclage de la solution d'échange comme décrit ci-dessus en utilisant un tel dispositif.

Un avantage supplémentaire de l'invention réside dans le fait qu'elle permet au patient initialement en dialyse péritonéale de conserver ses acquis (formation, qualité de vie) même si ses capacités rénales diminuent ou que son site d'injection/extraction n'est plus utilisable.

## Revendications

1. Dispositif d'épuration intra- et extracorporelle comprenant un réservoir de solution d'échange (11), un conduit d'introduction (5) de la solution d'échange connecté à une extrémité à ce réservoir (11) et des moyens de commande de débit (6) afin de gérer la circulation de la solution d'échange dans le conduit d'introduction (5) ainsi qu'un conduit d'évacuation (7) de la solution polluée, des moyens d'évacuation (13) de la solution polluée placés à une extrémité du conduit d'évacuation (7) et des moyens de commande de débit (8) afin de gérer la circulation de la solution polluée dans ledit conduit d'évacuation (7), **caractérisé par le fait que** le réservoir de solution d'échange (11) et les moyens d'évacuation (13) de la solution polluée sont aptes à être connectés soit directement sur un site du patient (P) à l'aide d'un élément spécifique (10) afin d'effectuer une dialyse péritonéale soit sur un circuit de circulation extracorporelle (1,2,3,4) qui est lui-même connecté au patient afin d'effectuer une épuration extracorporelle du sang, et **par le fait qu'**un module de commande (15) comprend des moyens de calcul (15b) qui pilotent les moyens de commande de débit (6) de la solution d'échange et les moyens de commande de débit (8) de la solution polluée afin de permettre un mode d'opération du dispositif pour la dialyse péritonéale et des moyens de calcul (15a) qui pilotent les moyens de commande de débit (6) de la solution d'échange, les moyens de commande de débit (8) de la solution polluée et la circulation dans le circuit extracorporel (1,2,3,4) afin de permettre un mode d'opération du dispositif pour l'épuration extracorporelle du sang.

2. Dispositif d'épuration intra- et extracorporelle selon la revendication 1, **caractérisé par le fait que** le dispositif comprend le circuit de circulation extracorporelle qui se compose d'un conduit d'extraction (1), d'un conduit de retour (3), des moyens d'épuration (4) situés entre le conduit d'extraction (1) et le conduit de retour (3) et des moyens de commande de débit (2) pour maintenir la circulation dans ce circuit.

3. Dispositif d'épuration intra- et extracorporelle selon la revendication 1 ou 2, **caractérisé par le fait que** le circuit de circulation extracorporelle (1,2,3,4) comprend également ledit élément spécifique (10) ou un élément à aiguille unique qui comportent au moins trois pièces de raccordement et permettent de connecter ledit circuit à un site unique du patient ainsi que sur les extrémités libres soit du conduit d'introduction (5) et du conduit d'évacuation (7), soit du circuit de circulation extracorporelle (1,2,3,4), ce circuit étant généralement apte à transporter du sang et de la solution d'échange dans et hors le corps par un site unique ainsi que par deux sites séparés du patient.

4. Dispositif d'épuration intra- et extracorporelle selon la revendication 2 ou 3, **caractérisé par le fait qu'**il comporte des moyens de contrôle (20, 21) placés sur les conduits (1, 3) du circuit de la circulation extracorporelle et destinés à assurer les cycles d'introduction/retour et d'extraction soit du sang soit de la solution d'échange ainsi qu'une chambre d'expansion (22) placée sur le conduit d'extraction (1) du circuit de la circulation extracorporelle qui peut se remplir lors du cycle d'extraction et se vider lors du cycle de retour.

5. Dispositif d'épuration extracorporelle selon l'une des revendications 3 à 4, **caractérisé par le fait que** les moyens de calcul (15a) sont adaptés à piloter la circulation sanguine dans le circuit extracorporel (1,2,3,4) à un débit inférieur à celui utilisé avec des accès vasculaires obtenus avec des cathéters ou fistules afin de réaliser un traitement de type ultrafiltration en extrayant le sang depuis un abord vasculaire périphérique unique et en retirant ensuite de l'eau du sang du patient à travers les moyens d'épuration (4).

6. Dispositif d'épuration intra- et extracorporelle selon l'une des revendications précédentes, **caractérisé par le fait que** le réservoir de solution d'échange (11) est relié aux moyens d'évacuation (13) de la solution polluée à l'aide d'un conduit de liaison (9) afin de permettre la re-circulation de la solution polluée.

7. Dispositif d'épuration intra- et extracorporelle selon l'une des revendications 1 à 5, **caractérisé par le fait que** le réservoir de solution d'échange (11) et les moyens d'évacuation (13) de la solution polluée sont formés par un composant unique afin de permettre la re-circulation de la solution polluée.

8. Dispositif d'épuration intra- et extracorporelle selon l'une des revendications précédentes, **caractérisé par le fait qu'**il comprend des moyens de fabrication en continu de la solution d'échange.

9. Dispositif d'épuration intra- et extracorporelle selon l'une des revendications précédentes, **caractérisé par le fait qu'**il comporte des moyens pour nettoyer les moyens d'épuration (4) ainsi que la tubulure, ces moyens de nettoyage permettant l'injection d'une substance nettoyante et d'une substance de rinçage et comportant de plus des moyens de contrôle et de sécurité.

## Claims

1. Device for intra- and extracorporeal purification comprising a reservoir for exchange solution (11), an admission conduit (5) for exchange solution connected at one end to this reservoir (11) and discharge control means (6) in order to manage the circulation of the exchange solution in the admission conduit (5) as well as an evacuation conduit (7) of polluted solution, evacuation means (13) for the polluted solution situated at one end of the evacuation conduit (7) and discharge control means (8) in order to manage the circulation of the polluted solution in said evacuation conduit (7), **characterised by** the fact that the reservoir for exchange solution (11) and the evacuation means (13) of the polluted solution are able to be connected either directly to a site of the patient (P) with the help of a specific element (10) in order to perform peritoneal dialysis or to a circuit for extracorporeal circulation (1,2,3,4) which is itself connected to the patient in order to perform extracorporeal blood purification, and by the fact that a control module (15) comprises computation means (15b) which control the discharge control means (6) of the exchange solution and the discharge control means (8) of the polluted solution in order to allow for an operation mode of the device for peritoneal dialysis and computation means (15a) which control the discharge control means (6) of the exchange solution, the discharge control means (8) of the polluted solution and the circulation in the extracorporeal circuit (1,2,3,4) in order to allow for an operation mode of the device for extracorporeal purification of blood.

2. Device for intra- and extracorporeal purification according to claim 1, **characterised by** the fact that the device comprises the circuit for extracorporeal circulation that is composed of an extraction conduit (1), a return conduit (3), purification means (4) situated between the extraction conduit (1) and the return conduit (3) and discharge control means (2) to maintain the circulation in this circuit.

3. Device for intra- and extracorporeal purification according to claim 1 or 2, **characterised by** the fact that the circuit for extracorporeal circulation (1,2,3,4) further comprises said specific element (10) or an element with a unique needle which comprise at least three connecting pieces and allow to connect said circuit to a unique site of the patient as well as to the free ends either of the admission conduit (5) and of the evacuation conduit (7) or of the circuit for extracorporeal circulation (1,2,3,4), this circuit being generally adapted to transport blood and exchange solution into and out of the body by a unique site as well as by two separated sites of the patient.

4. Device for intra- and extracorporeal purification according to claim 2 or 3, **characterised by** the fact that it comprises control means (20, 21) situated on the conduits (1, 3) of the circuit for extracorporeal circulation and adapted to assure the injection/return and extraction cycles either of blood or of exchange solution as well as an expansion chamber (22) situated on the extraction conduit (1) of the circuit for extracorporeal circulation which may be filled during the extraction cycle and emptied during the return cycle.

5. Device for extracorporeal purification according to any one of the claims 3 to 4, **characterised by** the fact that the computation means (15a) are adapted to control the blood circulation in the extracorporeal circuit (1,2,3,4) at a lower discharge with respect to the one used with vascular access sites obtained by catheters or fistulas in order to realise a treatment of the ultrafiltration type by extracting blood from a unique peripheral vascular access site and by then extracting water through purification means (4) out of the patient's blood.

6. Device for intra- and extracorporeal purification according to any one of the preceding claims, **characterised by** the fact that the reservoir for exchange solution (11) is connected to the evacuation means (13) of the polluted solution with the help of a connection conduit (9) in order to allow for re-circulation of the polluted solution.

7. Device for intra- and extracorporeal purification according to any one of the claims 1 to 5, **characterised by** the fact that the reservoir for exchange solution (11) and the evacuation means (13) of the polluted solution are formed by a single component in order to allow for re-circulation of the polluted solution.

8. Device for intra- and extracorporeal purification according to any one of the preceding claims, **characterised by** the fact that it comprises means for continuous production of exchange solution.

9. Device for intra- and extracorporeal purification according to any one of the preceding claims, **characterised by** the fact that it comprises means for cleaning the purification means (4) as well as the tubing, these cleaning means allowing the injection of a cleaning substance and of a rinsing substance and further comprising control and security means.

## Patentansprüche

1. Vorrichtung zur intra- und extrakorporalen Reinigung mit einem Vorratsgefäss (11) für Austauschlösung, einem mit einem Ende dieses Vorratsgefässes (11) verbundenen Einführungskanal (5) für die Austauschlösung und Mitteln (6) für die Durchflussregelung, um den Kreislauf der Austauschlösung im Einführungskanal (5) zu steuern, einem Ableitungskanal (7) für die verunreinigte Lösung, Mitteln (13) für die Ableitung der verunreinigten Lösung, die an einem Ende des Ableitungskanals (7) angeordnet sind, und Mitteln (8) für die Durchflussregelung, um den Kreislauf der verunreinigten Lösung im Ableitungskanal (7) zu steuern, **dadurch gekennzeichnet, dass** das Vorratsgefäss (11) für die Austauschlösung und die Mittel (13) für die Ableitung der verunreinigten Lösung entweder mit Hilfe eines speziellen Elements (10) direkt an eine Körperstelle des Patienten (P) angeschlossen werden können, um eine peritoneale Dialyse durchzuführen, oder an einen extrakorporalen Kreislauf (1, 2, 3, 4), der seinerseits an den Patienten angeschlossen ist, um eine extrakorporale Blutreinigung durchzuführen, und **dadurch**, dass ein Reglermodul (15) Rechnermittel (15b), die die Mittel (6) für die Durchflussregelung der Austauschlösung und die Mittel (8) für die Durchflussregelung der verunreinigten Lösung steuern, um einen Betriebsmodus der Vorrichtung für die peritoneale Dialyse zu ermöglichen, sowie Rechnermittel (15a) umfasst, die die Mittel (6) für die Durchflussregelung der Austauschlössung, die Mittel (8) für die Durchflussregelung der verunreinigten Lösung und die Zirkulation im extrakorporalen Kreis (1, 2, 3, 4) steuern, um einen Betriebsmodus der Vorrichtung für die extrakorporale Blutreinigung zu ermöglichen.

2. Vorrichtung zur intra- und extrakorporalen Reinigung nach Anspruch 1, **dadurch gekennzeichnet, dass** die Vorrichtung den extrakorporalen Kreislauf umfasst, der sich aus einem Entnahmekanal (1), einem Rücklaufkanal (3), zwischen dem Entnahmekanal (1) und dem Rücklaufkanal (3) befindlichen Reinigungsmitteln (4) sowie Mitteln (2) für die Durchflussregelung zusammensetzt, um die Zirkulation in diesem Kreis aufrecht zu erhalten:

3. Vorrichtung zur intra- und extrakorporalen Reinigung nach Anspruch 1 oder 2, **dadurch gekennzeichnet, dass** der extrakorporale Kreislauf (1, 2, 3, 4) ebenfalls das spezifische Element (10) oder ein Element mit nur einer Nadel umfasst, die mindestens drei Anschlussteile aufweisen und es gestatten, den Kreis an eine einzige Körperstelle des Patienten sowie an die freien Enden entweder des Einführungskanals (5) und des Ableitungskanals (7) oder aber des extrakorporalen Kreislaufs (1, 2, 3, 4) anzuschliessen, wobei dieser Kreis allgemein in der Lage ist, Blut und Austauschlösung über eine einzige Körperstelle ebenso wie über zwei getrennte Körperstellen des Patienten in den Körper hinein bzw. aus dem Körper heraus zu transportieren.

4. Vorrichtung zur intra- und extrakorporalen Reinigung nach Anspruch 2 oder 3, **dadurch gekennzeichnet, dass** sie Überwachungsmittel (20, 21), die an den Kanälen (1, 3) des extrakorporalen Kreislaufs angeordnet und dafür bestimmt sind, die Zyklen der Ein- und Rückführung bzw. der Entnahme des Blutes bzw. der Austauschlösung zu gewährleisten, sowie eine Expansionskammer (22) aufweist, die am Entnahmekanal (1) des extrakorporalen Kreislaufs angeordnet ist und sich während des Entnahmezyklus füllen, aber während des Rücklaufzyklus entleeren kann.

5. Vorrichtung zur extrakorporalen Reinigung nach einem der Ansprüche 3 bis 4, **dadurch gekennzeichnet, dass** die Rechnermittel (15a) in der Lage sind, die Blutzirkulation im extrakorporalen Kreis (1, 2, 3, 4) auf einen Durchfluss zu steuern, der niedriger als der mit Gefässzugängen benutzte ist, der mit Kathetern oder Fisteln erhalten wird, um eine Behandlung von der Art einer Ultrafiltration zu realisieren, indem das Blut von einem einzigen peripheren Gefässzugang entnommen und anschliessend das Wasser über die Reinigungsmittel (4) dem Blut des Patienten entzogen wird.

6. Vorrichtung zur intra- und extrakorporalen Reinigung nach einem der vorangehenden Ansprüche, **dadurch gekennzeichnet, dass** das Vorratsgefäss (11) für Austauschlösung mit Hilfe eines Verbindungskanals (9) an die Mittel (13) für die Ableitung der verunreinigten Lösung angeschlossen ist, um die wiederholte Zirkulation der verunreinigten Lösung zu ermöglichen.

7. Vorrichtung zur intra- und extrakorporalen Reinigung nach einem der Ansprüche 1 bis 5, **dadurch gekennzeichnet, dass** das Vorratsgefäss (11) für Austauschlösung und die Mittel (13) für die Ableitung der verunreinigten Lösung aus einem einzigen Bauteil bestehen, um die wiederholte Zirkulation der verunreinigten Lösung zu ermöglichen.

8. Vorrichtung zur intra- und extrakorporalen Reinigung nach einem der vorangehenden Ansprüche, **dadurch gekennzeichnet, dass** sie Mittel zur kontinuierlichen Herstellung der Austauschlösung umfasst.

9. Vorrichtung zur intra- und extrakorporalen Reinigung nach einem der vorangehenden Ansprüche, **dadurch gekennzeichnet, dass** sie Mittel zur Reinigung der Reinigungsmittel (4) sowie der Kanäle aufweist, wobei diese Mittel zur Reinigung das Einspritzen einer reinigenden Substanz und einer spülenden Substanz gestatten und darüber hinaus Mittel für die Überwachung und Sicherheit aufweisen.
